# EUROPÄISCHE PATENTSCHRIFT

(11) **EP 0 839 153 B1**
(45) Veröffentlichungstag und Bekanntmachung des Hinweises auf die Patenterteilung: **16.08.2001**
(21) Anmeldenummer: 96923933.4
(22) Anmeldetag: 26.06.1996
(51) Int. Cl.: C07K 1/06, C12P 21/02

(54) **VERFAHREN ZUR HERSTELLUNG VON PEPTIDEN UND N-CARBAMOYL-GESCHÜTZTEN PEPTIDEN**
PROCESS OF PREPARATION OF PEPTIDES AND N-CARBAMOYL PROTECTED PEPTIDES
PROCEDE DE PRODUCTION DE PEPTIDES ET PEPTIDES A PROTECTION N-CARBAMOYLE

(30) Priorität: 11.07.1995 DE 19524710; 05.02.1996 DE 19603844; 26.02.1996 DE 19607100
(43) Veröffentlichungstag der Anmeldung: 06.05.1998
(60) Teilanmeldung: 01102668.9
(73) Patentinhaber: Degussa AG, 40474 Düsseldorf (DE)
(72) Erfinder: BOMMARIUS, Andreas, D-60323 Frankfurt (DE); DRAUZ, Karlheinz, D-63579 Freigericht (DE); EICHHORN, Uwe, D-01454 Grosserkammsdorf (DE); JAKUBKE, Hans-Dieter, D-04279 Leipzig (DE); KOTTENHAHN, Matthias, D-63579 Freigericht (DE)
(86) Internationale Anmeldenummer: EP9602782
(87) Internationale Veröffentlichungsnummer: WO9703091

(56) Entgegenhaltungen:
- EP-A- 0 625 571
- WO-A-88/01650
- WO-A-94/00577
- DE-A- 3 607 194
- DE-C- 4 214 157
- CHEMICAL ABSTRACTS, vol. 76, no. 17, 24.April 1972 Columbus, Ohio, US; abstract no. 100035, PAVARS, A. ET AL: "Synthesis of N-carbamoylated fragments of [5-valine]-angiotensin II" XP002020877 & ZH. OBSHCH. KHIM. (1971), 41(10), 2312-17 CODEN: ZOKHA4, 1971,
- CHEMICAL ABSTRACTS, vol. 75, no. 3, 19.Juli 1971 Columbus, Ohio, US; abstract no. 20982, SMIRNOV, O. V. ET AL: "Synthesis of some C-terminal tetrapeptide derivatives of the hormone gastrin" XP002020878 & KHIM. PRIR. SOEDIN. (1971), 7(1), 94-7 CODEN: KPSUAR, 1971,
- CHEMICAL ABSTRACTS, vol. 84, no. 17, 26.April 1976 Columbus, Ohio, US; abstract no. 122296, ROMANOVSKAYA, I. K. ET AL: "Solid-phase synthesis of (1-asparagine, 5-valine, 8-alanine)- and (1-hydantoic acid, 5-valine, 8-alanine)-angiotensin II" XP002020879 & BIOORG. KHIM. (1975), 1(9), 1257-62 CODEN: BIKHD7, 1975,
- HELVETICA CHIMICA ACTA, Bd. 58, Nr. 1, 29.Januar 1975, BASEL CH, Seiten 139-146, XP002020874 H. KÜNZI UND R.O. STUDER: "Zur Verwendung des Tosylaminocarbonyl-Restes als Amino-Schutzgruppe in der Peptidchemie"
- JOURNAL OF AGRICULTURAL AND FOOD CHEMISTRY, Bd. 42, Nr. 7, Juli 1994, WASHINGTON US, Seiten 1397-1401, XP002020875 N.M. HOOPER ET AL.: "Stability of N-Derivatized and alpha-methyl Analogues of Aspartame to Hydrolysis by Mammalian Cell-Surface Peptidases"
- "The Merck Index, 11th Edition" 1989 , MERCK & CO., INC. , RAHWAY, NY, US XP002020876 569 see No. 861, Aspartame on page 132

## Beschreibung

Die Erfindung betrifft ein Verfahren zur enzymatischen Herstellung von geschützten Di- oder Oligopeptiden und die Abspaltung der verwendeten Schutzgruppe.

Synthetische kurzkettige Peptide finden in der Pharmakologie und in der parenteralen Ernährung zunehmend Verwendung. Als Beispiel für ein pharmakologisch wirksames Dipeptid sei Kyotorphin (L-Tyr-L-Arg) genannt, das die Freisetzung von Enkephalinen fördert, endogenen Substanzen also, die im Gehirn eine analgetische und beruhigende Wirkung besitzen (Hughes, 1975).

Die enzymatische Herstellung von Di- oder Oligopeptiden ist bereits bekannt (Helv. Chim. Acta, Vol. 58(18), S. 139-46, 1975; CA 79:100035; CA 75:20982; CA 84:122296) und bedient sich generell der Schutzgruppentechnologie. So wird in US-A-571,037 die Verwendung einer Formylschutzgruppe, in JP 62074296 einer Acetylschutzgruppe, in US-A-4,935,355 einer Benzylschutzgruppe oder in Tetrahedron 1992, 48, 1115 einer Phenacetylschutzgruppe beschrieben. Diese Varianten haben den Nachteil, daß die Schutzgruppen z. T. nicht kostengünstig sind (Benzyl, Phenacetyl), nur schwer wieder abspaltbar (Acetyl, Formyl) oder nur unter ganz bestimmten Bedingungen (Hydrogenolyse) entfernt werden können (Benzyl).

Es ist prinzipiell bekannt, eine Carbamoylschutzgruppe von Aminosäuren enzymatisch zu entfernen (WO-A-9400577; EP-A-625571).

Aufgabe der Erfindung war daher, ein Verfahren zur Synthese von Peptiden zu entwickeln, das besonders einfach und kostengünstig ist, eine einfache und schonende Abspaltung der Schutzgruppe ermöglicht sowie eine einfache Aufarbeitung und Abtrennung des Enzyms ermöglicht.

Diese Aufgabe der Erfindung zur Herstellung eines Peptides der allgemeinen Formel I 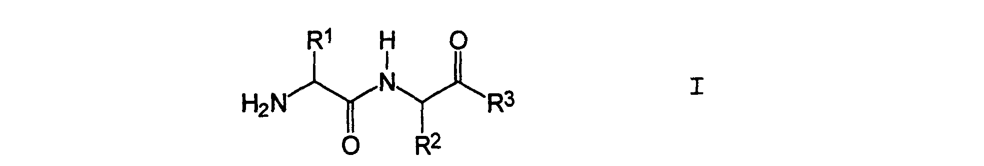 worin
- R¹ und R²: unabhängig voneinander Wasserstoff, (C₁-C₆) Alkyl, das ggf. ein oder mehrfach mit Heteroatome, wie N, O oder S, unterbrochen oder substituiert sein kann, wobei die Heteroatome ihrerseits mit Wasserstoff,
(C₁-C₄) Alkyl oder Benzyl substituiert sein können oder über eine Doppelbindung mit der Alkylgruppe verbunden sein können, Phenyl oder Benzyl, die beide ggf. ein- oder mehrfach mit Halogen oder Hydroxy substituiert sein können, Heteroaralkyl, wie 3-Indolylmethyl, 2-, 3- oder 4-Pyridylmethyl,
- R³: (C₁-C₄) Alkoxy, NH₂, Hydroxy, NF¹R², Benzyloxy, das ggf. ein- oder mehrfach durch Halogen, Nitro, NH₂, (C₁-C₄) Alkyl, (C₁-C₄) Alkoxy substituiert sein kann, oder eine oder mehrere Einheiten des Typs II 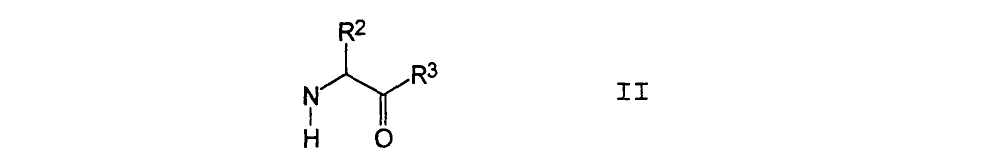 bedeuten, wird dadurch gelöst, daß man Verbindungen des Typs III oder eine Salzform davon, 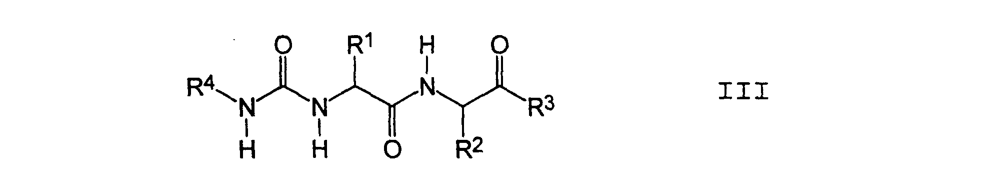 worin R¹, R² und R³ die oben angegebene Bedeutung besitzen und
- R⁴: Wasserstoff, (C₁-C₄) Alkyl, Phenyl, das ggf. ein- oder mehrfach mit Halogen, (C₁-C₄) Alkyl, (C₁-C₄) Alkoxy, Nitro, CN, CF₃, (C₁-C₆) Alkoxycarbonyl, COOH oder -NR¹R² substituiert sein kann, Aralkyl, wie Benzyl, das seinerseits mit Halogen, (C₁-C₄) Alkyl oder (C₁-C₄) Alkoxy substituiert sein kann, Naphthyl, Heteroaralkyl, wie 2-, 3- oder 4-Thienyl, 2-, 3- oder 4-Pyridyl oder 2-Chinolyl
bedeutet,
zur Abspaltung der Carbamoyl-Schutzgruppe mit einer Carbamoylase, wahlweise in Gegenwart eines Lösungsmittels, zur Reaktion.

Weiterhin wurde gefunden, daß man carbamoylgeschützte Peptide der allgemeinen Struktur III, 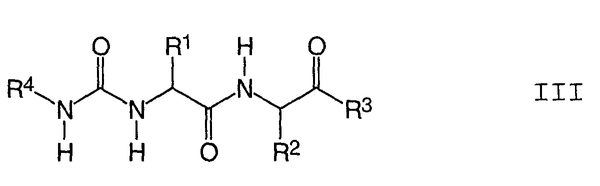 worin
- R¹ und R²: unabhängig voneinander Wasserstoff, (C₁-C₆) Alkyl, das ggf. ein oder mehrfach mit Heteroatome, wie N, O oder S, unterbrochen oder substituiert sein kann, wobei die Heteroatome ihrerseits mit Wasserstoff, (C₁-C₄) Alkyl oder Benzyl substituiert sein können oder über eine Doppelbindung mit der Alkylgruppe verbunden sein können, Phenyl oder Benzyl, die beide ggf. ein- oder mehrfach mit Halogen oder Hydroxy substituiert sein können, Heteroaralkyl, wie 3-Indolylmethyl, 2-, 3- oder 4-Pyridylmethyl,
- R³: (C₁-C₄) Alkoxy, NH₂, Hydroxy, NR¹R², Benzyloxy, das ggf. ein- oder mehrfach durch Halogen, Nitro, NH₂, (C₁-C₄) Alkyl, (C₁-C₄) Alkoxy substituiert sein kann,
- R⁴: Wasserstoff, (C₁-C₄) Alkyl, Phenyl, das ggf. ein- oder mehrfach mit Halogen, (C₁-C₄) Alkyl, (C₁-C₄) Alkoxy, Nitro, CN, CF₃, (C₁-C₆) Alkoxycarbonyl, COOH oder -NR¹R² substituiert sein kann, Aralkyl, wie Benzyl, das seinerseits mit Halogen, (C₁-C₄) Alkyl oder (C₁-C₄) Alkoxy substituiert sein kann, Naphthyl, Heteroaralkyl, wie 2-, 3- oder 4-Thienyl, 2-, 3- oder 4-Pyridyl oder 2-Chinolyl
bedeuten, erhält, indem man aus einer Verbindung des Typs IV oder eine Salzform von IV, in der R¹ und R⁴ die oben angegebene Bedeutung besitzen, 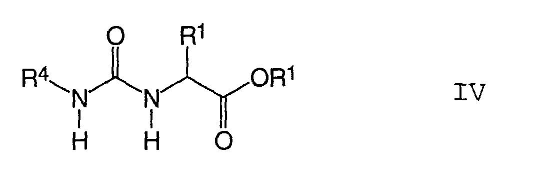 in Gegenwart von Hydrolasen, wahlweise in Gegenwart eines Lösungsmittels und wahlweise in Gegenwart einer Base, und einer Verbindung des Typs V, oder eines Säureadditionssalzes hiervon, worin R² und R³ die oben angegebene Bedeutung besitzen, 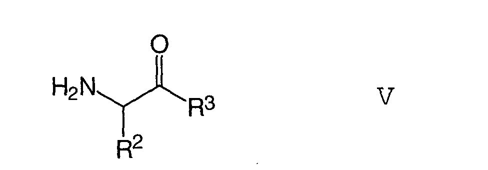 herstellt.

Unter der Bezeichnung "Alkylgruppen" sind sowohl "geradkettige" als auch "verzweigte" Alkylgruppen zu verstehen.

Unter der Bezeichnung "geradkettige Alkylgruppe" sind beispielsweise Reste wie Methyl, Ethyl, n-Propyl, n-Butyl, n-Pentyl, n-Hexyl, unter "verzweigter Alkylgruppe" Reste wie beispielsweise Isopropyl oder tert.-Butyl zu verstehen.

Die Bezeichnung Halogen steht für Fluor, Chlor, Brom oder Jod. Die Bezeichnung "Alkoxygruppe" stellt Reste wie beispielsweise Methoxy, Ethoxy, Propoxy, Butoxy, Isopropoxy, Isobutoxy oder Pentoxy dar.

Bevorzugt können mit diesem Verfahren Dipeptide, wie beispielsweise Aspartam hergestellt werden.

Ein weiterer Vorteil des Verfahrens ist, daß man bei der Peptidkopplung in hochdichter Suspension arbeiten kann, wobei Substrat und Produkt in teilweiser fester Form vorliegen können.

Das neue Verfahren zur Synthese von Peptiden unter Verwendung der beschriebenen Carbamoyl-geschützten Peptide als Zwischenprodukt, beinhaltet drei Reaktionsschritte.
A) Darstellung der N-Carbamoyl-Aminosäure bzw. des N-Carbamoyl-Aminosäurederivates
B) Knüpfung der Peptidbindung zwischen Carbamoylgeschütztem Elektrophil und Nukleophil
C) Abspaltung der Carbamoyl-Schutzgruppe

Die Darstellung der N-Carbamoyl-Aminosäure bzw. des N-Carbamoyl-Aminosäurederivates kann in an sich literaturbekannter Weise durchgeführt werden. Vorzugsweise erfolgt die Umsetzung der Aminosäure bzw. des Aminosäurederivates mit einem Isocyanat der allgemeinen Struktur VI,

R¹―NCO VI

wobei R¹ die oben angegebene Bedeutung hat, in einem Zweiphasensystem aus H₂O/organischem Lösungsmittel, wobei das organische Lösungsmittel beispielsweise Toluol, Chlorbenzol oder tert.Butylmethylether sein kann. Die Umsetzung erfolgt bei Temperaturen zwischen 0 °C und 120 °C, vorzugsweise zwischen 20 °C und 70 °C. Gegebenenfalls kann dem Reaktionsgemisch eine anorganische Base, wie beispielsweise NaOH, KOH, K₂CO₃, Na₂CO_{3,} Kaliumhydrogencarbonat oder Natriumhydrogencarbonat oder eine organische Base, wie beispielsweise Triethylamin oder Tributylamin zugesetzt werden. Das Phenylisocyanat der Struktur III wird vorteilhafterweise im geringen Überschuß verwendet.

Besonders bevorzugt sind Umsetzungen zu den N-Carbamoylgeschützten Aminosäuren bzw. Aminosäurederivaten mit einem Cyanat der Struktur VII,

M^{⊕ ⊖}OCN VII

wobei M^{⊕} für Na^{⊕}, K^{⊕}, Ag^{⊕}, Pb^{⊕} (OCN) oder Ammoniumionen, wie beispielsweise Tetraethylammonium, Tetrabutylammonium oder Benzyltriethylammonium, steht.

Die Umsetzung erfolgt in an sich bekannter Weise in wäßriger Lösung bei Temperaturen zwischen 0 °C - 120 °C, vorzugsweise zwischen 60 °C - 100 °C und ggf. unter Zusatz einer anorganischen Base, wie beispielsweise NaOH, KOH, K₂CO₃ oder Na₂CO₃. Als günstig erweist sich, wenn die Reaktion in Gegenwart von leichten Überschüssen von 1.01 - 2 Äquivalenten, vorzugsweise 1.1 - 1.2 Äquivalenten an Cyanat durchgeführt wird.

Die Knüpfung der Peptidbindung wird in an sich bekannter Weise mit Hilfe einer Hydrolase durchgeführt (Jakubke, Kuhl und Könnecke; Angew. Chem. 1985, Vol. 97, S. 79 - 87). Dabei ist es für die Reaktionsbedingungen unerheblich, ob die Peptidknüpfung kinetisch oder thermodynamisch kontrolliert abläuft. Das erfindungsgemäße Verfahren ist auf beide Verfahrensvarianten anwendbar. Unter der Bezeichnung "Salzform" des Verbindungstyps IV sind allgemein ionische Strukturen zu verstehen. So kann ein mögliches Kation, wie beispielsweise Na^{⊕}, K^{⊕}, Ag^{⊕}, Pb^{2⊕} oder ein Ammoniumion, wie beispielsweise Tetraethylammonium, Tetrabutylammonium oder Benzyltriethylammonium, eine negative Ladung am Verbindungstyp IV neutralisieren. Die negative Ladung kann dabei über das Molekül (Verbindungstyp IV) verteilt sein oder an einem Stickstoffatom oder der Carboxylgruppe in Form einer Carboxylatgruppe lokalisiert sein.

Überraschenderweise verläuft diese Umsetzung mit der für diese Reaktionssequenz neuen Carbamoylgruppe als Schutzgruppe in guten Raum-/Zeitausbeuten und ermöglicht eine einfache Isolation der Produkte. Ebenso überraschend ist breite Anwendbarkeit der Reaktion.

Unter einem Säureadditionssalz von V ist beispielsweise ein HCl-Salz, HBr-Salz oder ein H₂SO₄-Salz zu verstehen.

Die Abspaltung der Carbamoyl-Schutzgruppe erfolgt beispielsweise unter milden Reaktionsbedingungen mit Hilfe einer Carbamoylase. Diese Art der Abspaltung von Carbamoylgruppen von Peptiden ist neu. Sie ist deswegen besonders vorteilhaft, weil sie neben dem Enzym nur Wasser als Einsatzstoff zur Spaltung benötigt und weil als Spaltprodukte neben dem Peptid nur Kohlendioxid und Ammoniak entstehen, von denen sich das Peptid sehr leicht abtrennen läßt. Die Carbamoylase kann dazu ggf. in teilgereinigtem, gereinigtem, isoliertem oder immobilisiertem Zustand eingesetzt werden. Die Reaktion ist irreversibel, und es werden bis zu 100 % Umsatz beobachtet. Die Umsetzung erfolgt in wäßriger Lösung bei Temperaturen zwischen 10 - 50 °C, vorzugsweise 20 - 35 °C, ganz besonders bevorzugt 25 - 30 °C, und pH-Werten zwischen 5 und 11, bevorzugt 6,5 - 8,5 und ganz besonders bevorzugt zwischen 7 - 8.

Bei der Hydrolyse mit Carbamoylase entstehen Kohlendioxid und Ammoniak, die ggf. aus der Reaktionsmischung durch Ausgasung entweichen.

Aus der mit Carbamoylase behandelten Peptidlösung kann das nun ungeschützte Peptid durch Kristallisation mittels Eindampfung, Kühlung oder ggf. Zusatz von organischen Lösungsmitteln ausgefällt und isoliert werden.

Als geeignet erwiesen sich Carbamoylasen, die bei der Deutschen Sammlung für Mikroorganismen unter folgenden Nummern hinterlegt sind: DSM 7329, DSM 7330, DSM 9771. Das neue Verfahren schließt aber andere Mikroorganismen als die obengenannten nicht aus.

Die Abspaltung der Carbamoylschutzgruppe kann analog oben beschrieben auch in Gegenwart eines zusätzlichen Enzyms (neben den genannten Carbamoylasen), wie beispielsweise Thermolysin, erfolgen.

Das neue Verfahren wird anhand der folgenden Ausführungsbeispiele näher erläutert, ist aber nicht auf diese beschränkt.

Das neue Verfahren wird anhand der folgenden Ausführungsbeispiele näher erläutert, ist aber nicht auf diese beschränkt.

### Beispiel 1

### Exemplarische Darstellung von N-Carbamoyl-Aminosäuren und Derivaten am Beispiel der N-Carbamoyl-L-Asparaginsäure

12 g (0,3 mol) NaOH-Plätzchen werden in ca. 200 ml H₂O vorgelegt und gelöst. Die Asparaginsäure (33,3 g, 0,25 mol) wird in 50 ml Wasser gelöst zugegeben und das Reaktionsgemisch auf 80 °C erwärmt. Der pH-Wert liegt bei 8.7. Innerhalb von 5 min wird 16,6 g (0,255 mol) Natriumcyanat zugegeben (pH-Wert steigt auf 9.3, T auf 86 ° C). Nach 1 h Reaktionszeit ist keine Asparaginsäure mehr nachzuweisen.

### Beispiele zur Synthese der Peptide:

Die Aminosäuren und Peptide werden nach den international gültigen Regeln abgekürzt (IUPAC-JUB Joint Commission on Biochemical Nomenclature (JCBN); Nomenclature and Symbolism for Amino Acids and Peptides. Eur. J. Biochem. 158, 9 - 37 (1984)).
AC-: Aminocarbonyl- (Carbamoyl-, NH₂CO-)

### Beispiel 2: Synthese von AC-Asp-Phe-OMe

50 mg (0,2 mmol) AC-Asp(OK)-OK und 83 mg (0,4 mmol) H-Phe-OME•HCl werden in einem verschließbaren Polypropylengefäß mit 80 µl Puffer (0,5 M Hepes/Na⁺; pH 7) versetzt und sofort intensiv mit einem Spatel durchmischt. Der Reaktionsansatz wird in einem Wasserbad auf 40 °C thermostatiert und die Reaktion anschließend mit 20 µl Puffer, die 4 mg Thermolysinpräparation (Sigma P 1512) enthalten, gestartet. Nach 1 h, 2,5 h, 16 h, 22 h, 29 h und 39 h werden Proben entnommen und in 0,75 ml 4 %iger wäßriger Essigsäure abgestoppt. Die analytische Auswertung erfolgt hier wie in den folgenden Beispielen mittels HPLC und Vergleich mit einer authentischen Probe.

### Ausbeute:

| Zeit (h) | Ausbeute (% d. Th.) | H-Phe-OH/H-Phe-OMe(t=0) (-/-) |
|---|---|---|
| 1 | 7 | 0,023 |
| 2,5 | 14 | 0,030 |
| 16 | 55 | 0,083 |
| 22 | 69 | 0,119 |
| 29 | 77 | 0,12 |
| 39 | 81 | 0,125 |

### Beispiel 3 a - e

Analog Beispiel 2 werden statt 80 µl Puffer 120 (a), 160 (b), 200 (c), 240 (d) und 280 µl Puffer zum Suspendieren der Reaktanden verwendet. Die Probennahme erfolgt nach 29 bzw. 39 h.

### Ausbeute:

| Biespiel | Ausbeute (% d. Th.) | | H-Phe-OH/H-Phe-OMe(t=0) (-/-) | |
|---|---|---|---|---|
| | 29 h | 39 h | 29 h | 39 h |
| 3a | 79 | 82 | 0,139 | 0,136 |
| 3b | 79 | 76 | 0,144 | 0,165 |
| 3c | 73 | 75 | 0,169 | 0,169 |
| 3d | 64 | 64 | 0,216 | 0,22 |
| 3e | 59 | 61 | 0,215 | 0,224 |

### Beispiel 4

44 mg (0,175 mmol) AC-Asp(OK)-OK und 41,5 mg (0,2 mmol) H-Phe-OMe•HCl werden in einem verschließbaren Polypropylengefäß mit 80 µl Puffer (0,5 M Hepes/Na⁺; pH 7) versetzt und sofort intensiv mit einem Spatel suspendiert. Nach der Überführung des Reaktionsgefäßes in ein auf 40 °C thermostatiertes Wasserbad wird die Reaktion durch Zugabe von 15 µl Puffer, die 1,5 mg Thermolysinpräparation (Sigma P 1512) enthalten, gestartet. Nach 1 h, 2,5 h, 5 h, 6,5 h und 22 h werden Proben entnommen und in 0,75 ml 4 %iger wäßriger Essigsäure abgestoppt und mittels HPLC analysiert.

### Ausbeute:

| Zeit (h) | Ausbeute (% d. Th.) | H-Phe-OH/H-Phe-OMe(t=0) (-/-) |
|---|---|---|
| 1 | 7 | 0,023 |
| 2,5 | 16 | 0,039 |
| 5 | 23 | 0,064 |
| 6,5 | 28 | 0,078 |
| 22 | 48 | 0,136 |

### Beispiel 5

Analog Beispiel 4 werden die Reaktanden statt mit 80 µl

Puffer mit 10 (a), 30 (b), 50 (c), 130 (d) und 180 µl (e) Puffer versetzt. Die Probennahme erfolgt nach 22 h.

### Ausbeute:

| Beispiel | Ausbeute (% d. Th.) | H-Phe-OH/H-Phe-OMe(t=0) (-/-) |
|---|---|---|
| 5a | 7 | 0,034 |
| 5b | 31 | 0,072 |
| 5c | 29 | 0,105 |
| 5d | 39 | 0,319 |
| 5e | 41 | 0,249 |

### Beispiel 6: Synthese von AC-Asp-Phe-NH₂

250 mg (1.0 mmol) AC-Asp(OK)-OK und 400 mg (2.0 mmol) H-Phe-NH₂•HCl werden in einem verschließbaren Polypropylengefäß mit 400 µl Puffer (0,5 M Hepes/Na⁺; ph 7) versetzt und sofort intensiv mit einem Spatel suspendiert. Nach der Überführung des Reaktionsgefäßes in ein auf 40 °C thermostatiertes Wasserbad wird die Reaktion durch Zugabe von 50 µl Puffer, die 10 mg Thermolysinpräparation (Sigma P 1512) enthalten, gestartet. Nach 44 h werden Proben entnommen und in 0,75 ml 4 %iger wäßriger Essigsäure abgestoppt und mittels HPLC analysiert. Die Reaktion wird durch Zugabe von 1 ml 1 N wäßrige HCl beendet und über Nacht bei 4 °C belassen. Anschließend wird der Niederschlag abgesaugt und mit eiskaltem Wasser gewaschen.
Ausbeute: 62 % d. Th.
Schmelzbereich: 221 - 223 °C
¹H-NMR (D₆-DMSO, 300 MHz): δ=2,38-2,6 (m, 2 H, -^{β}CH₂-) , δ=2,8-2,88 (m, 1 H, -^{β}CH₂-) , δ=5,72 (s, 2 H, -NH₂), δ=6,30 (d, 1 H, -^{α}NH-, J = 7,69), δ=7,l3 (s, 1 H, Phe-NH₂) , δ=7,26-7,28 (m, 5 H, Phe-C₆H₅), δ=7,33 (s, 1 H, Phe-NH₂), δ=7,83 (d, 1 H, -^{α}NH-, J = 8,4)

### Beispiel 7: Synthese von AC-Asp-Phe-Leu-NH₂

67,4 mg (0,2 mmol) AC-Asp-Phe-OMe und 26 mg (0,2 mmol) H-Leu-NH₂ (Bachem Biochemica GmbH) werden in einem Polypropylenreaktionsgefäß in 180 µl Puffer (0,5 M Hepes/Na⁺, pH 7,9) gelöst. Nach Zugabe von 10 µl 10 M wäßriger NaOH wird die Reaktion mit 10 µl α-Chymotrypsinlösung (10 mg/ml Puffer; Serva, 3 x krist.) gestartet. Nach 2, 5, 10, 20, 30 und 60 min werden Proben entnommen, in 1 ml 4 %iger wäßriger Essigsäure gelöst und mittels HPLC analysiert. Der verbliebene Reaktionsansatz wird in 1 ml Methanol aufgenommen , filtriert und das Filtrat mit 1 ml 1 N wäßriger HCl und 4 ml Wasser versetzt. AC-Asp-Phe-Leu-NH₂ kristallisierte innerhalb von 48 h aus. Die Kristalle wurden abgetrennt und 2 mal mit eiskaltem Wasser (je ca. 1,5 ml) gewaschen und anschließend über P₄O₁₀ im Vakuum getrocknet.

### Ausbeute:

| Zeit (min) | AC-Asp-Phe-OH (% d. Th) | AC-Asp-Phe-Leu-NH₂ (% d. Th.) |
|---|---|---|
| 2 | 18,3 | 11,6 |
| 5 | 19 | 23,9 |
| 10 | 19,8 | 41,5 |
| 20 | 21,2 | 60,5 |
| 30 | 21,6 | 70,2 |
| 60 | 20,8 | 79,1 |

¹H-NMR;COSY(DMSO, 300 MHz): δ=0,82 (d, 3 H, Leu-^{δ}CH₃), δ=0,87 (d, 3 H, Leu-^{δ} CH₃), δ=1,41-1,6 (m, 3 H, Leu-^{γ}CH-, Leu-^{β} CH₂-), δ=2,35-2,58 (m, 2 H, Asp-^{β}CH₂-) , δ=2,81-3,06 (m, 2 H, Phe-^{β}CH₂-), δ=4,18 (dt, 1 H, Leu-^{α}CH-) , δ=4,33 (dt, 1 H, Asp-^{α} CH-), δ=4,44 (dt, 1 H, Phe-^{α}CH-), δ=5,70 (s, 2 H, AC-NH₂), δ=6,30 (d, 1 H, Asp-^{α}NH-), δ=6,96 (s, 1 H, Leu-NH₂), δ=7,05 (s, 1 H, Leu-NH₂), δ=7,14-7,27 (m, 5 H, Phe-C₆H₅), δ=7,85-7,96 (m, 2 H, Leu-^{α}NH, Phe-^{α}NH)

### Beispiel 8: Synthese von AC-Asp-Phe-Ile-Gly-OMe

Analog Beispiel 7 werden statt H-Leu-NH₂ 0,2 mmol (47,8 mg) H-Ile-Gly-OMe•HCl eingesetzt. Zur pH-Wert-Einstellung werden 35 µl des Puffers durch 10 M wäßrige NaOH ersetzt.

### Ausbeute nach 70 min:

| AC-Asp-Phe-OH (% d. Th.) | AC-Asp-Phe-Ile-Gly-OH (% d. Th.) | AC-Asp-Phe-Ile-Gly-OMe (% d. Th.) |
|---|---|---|
| 28,4 | 3,9 | 67,7 |

¹H-NMR;COSY(DMSO, 300 MHz): δ=0,77-0,88 (m, 6 H, Ile-^{δ}CH₃), δ=1,00-1,15 (m, 1 H, Ile-^{γ}CH₂-), δ=1,38-1,52 (m, 1 H, Ile-^{γ}CH₂-), δ=1,65-1,78 (m, 1 H, Ile-^{β}CH-), δ=2,34-2,58 (m, 2 H, Asp-^{β}CH₂-), δ=2,78-2,88 (m, 1 H, Phe-^{β}CH₂-), δ=2,94-3,2 (m, 1 H, Phe-^{β}CH₂-), δ=3,62 (s, 3 H, OCH₃), δ=3,75-3,95 (m, 2 H, Gly-^{α}CH₂-), δ=4,17 (t, 1 H, Ile-^{α}CH-), δ=4,30-4,40 (m, 1 H, Asp-^{α}CH-), δ=4,48-4,58 (m, 1 H, Phe-^{α}CH-), δ=5,7 (s-breit, AC-NH₂), δ=6,30 (d, 1 H, Asp-^{α}NH-), δ=7,12-7,26 (m, 5 H, Phe-C₆H₅-), δ=7,80 (d, 1 H, Phe-^{α}NH-), δ=7,92 (d, 1 H, Leu-^{α}NH-), δ=8,32 (t, 1 H, Gly-^{α}NH-).

### Beispiel 9: Synthese von AC-Tyr-Ile-Ala-NH₂

a) 44,8 mg (0,2 mmol) AC-Tyr-OH und 47,4 mg (0,2 mmol) H-Ile-Ala-NH₂•HCl werden in einem verschließbaren Polypropylengefäß mit 45 µl Puffer (0,5 M Hepes/Na⁺; pH 7) versetzt und sofort intensiv mit einem Spatel suspendiert. Die pH-Wert-Einstellung erfolgt durch Zugabe von 25 µl 10 M wäßriger NaOH. Nach der Überführung des Reaktionsgefäßes in ein auf 40 °C thermostatiertes Wasserbad wird die Reaktion durch Zugabe von 10 µl Puffer, die 1 mg Thermolysinpräparation (Sigma P 1512) enthalten, gestartet. Nach 3 h wird eine Probe entnommen und in 1 ml 4 %iger wäßriger Essigsäure und 200 µl Acetonitril abgestoppt und mittels HPLC analysiert. Die Aufarbeitung des verbliebenen Reaktionsansatzes erfolgt wie in Beispiel 7 mit dem Unterschied, daß statt 1 N HCl 4 %ige wäßrige Essigsäure verwendet wird.
   Ausbeute an AC-Tyr-Ile-Ala-NH₂ nach 3 h: 76 % d. Th.
   ¹H-NMR;COSY(DMSO, 300 MHz): δ=0,76-0,84 (m, 6 H, Ile-^{δ} CH₃, Ile-^{γ}CH₃), δ=1.00-1,12 (m, 1 H, Ile-^{γ}CH₂-), δ=1,22 (d, 3 H, Ala-^{β}CH₃), δ=1,36-1,42 (m, 1 H, Ile-^{γ}CH₂-), δ=1.68-1,78 (m, 1 H, Ile-^{β}CH-), δ=2,56-2,68 (m, 1 H, Tyr-^{β}CH₂-), δ=2,80-2,90 (m, 1 H, Tyr-^{β}CH₂-), δ=4,12-4,24 (m, 2 H, Ala-^{α}CH-, Ile-^{α} CH-), δ=4,28-4,38 (m, 1 H, Tyr-^{α}CH-), δ=5,58 (s, 2 H, AC-NH₂), δ=6,03 (d, 1 H, Tyr-^{α}NH-), δ=6,61 (d, 2 H, Tyr-ArH), δ=6,92-6,98 (m, 3 H, 2 Tyr-ArH, 1 Ala-NH₂), δ=7,20 (s, 1 H, Ala-NH₂), δ=7,84 (d, 1 H, Ala-^{α}NH-), δ=7,92 (d, 1 H, Ile-^{α}NH-), δ=9,12 (s, 1 H, Tyr-OH)
b) Analog Beispiel 9a werden 224 mg (1.0 mmol) AC-Tyr-OH, 237,7 mg (1,0 mmol) H-Ile-Ala-NH₂•HCl, 225 µl Puffer, 125 µl 10 M NaOH und 50 µl Thermolysinsuspension, die 5 mg Thermolysin (Sigma P1512) enthalten, eingesetzt.
   Ausbeute an AC-Tyr-Ile-Ala-NH₂ nach 16 h:
   228 mg (= 56 % d. Th.)

### Beispiel 10: Synthese von AC-Tyr-Val-NH₂

Analog Beispiel 9a werden statt H-Ile-Ala-NH₂•HCl 31,2 mg (0,2 mmol) H-Val-NH₂•HCl eingesetzt.
Ausbeute an AC-Tyr-Val-NH₂ nach 18 h: 65 % d. Th.
¹H-NMR (D₆-DMSO, 300 MHz): δ=0,78-0,88 (m, 6 H, Val-^{γ}CH₃), δ=1,92-2,00 (m, 1 H, Val-^{β}CH-), δ=2,58-2,68 (m, 1 H, Tyr-^{β}CH₂-), δ=2,80-2,90 (m, 1 H, Tyr-^{β}CH₂-), δ=4,11 (dd, 1 H, Val-^{α}CH-), δ=4,28-4,38 (m, 1 H, Tyr-^{α}CH-) , δ=5,57 (s, 2 H, AC-NH₂), δ=6,08 (d, 1 H, Tyr-^{α}NH-), δ=6,62 (d, 2 H, Tyr-ArH), δ=6,97 (d, 2 H, Tyr-ArH), δ=7,03 (s, 1 H, Val-NH₂), δ=7,31 (s, 1 H, Val-NH₂), δ=7,67 (d, 1 H, Val-^{α}NH-), δ=9,12 (s, 1 H, Tyr-OH)

### Beispiel 11: Synthese von AC-Tyr-Met-NH₂

Analog Beispiel 9a werden statt H-Ile-Ala-NH₂•HCl 37 mg (0,2 mmol) H-Met-NH₂•HCl eingesetzt.
Ausbeute an AC-Tyr-Met-NH₂ nach 14 h: 75 % d. Th.
¹H-NMR (D₆-DMSO, 300 MHz): δ=1,75-1,85 (m, 1 H, Met-^{β}CH₂-), δ=1,88-2,00 (m, 1 H, Met-^{β}CH₂-), δ=2,02 (s, 3 H, Met-S-CH₃), δ=2,30-2,45 (m, 2 H, Met-^{β}CH₂-), δ=2,60-2,85 (m, 2 H, Tyr-^{β}CH₂-), δ=4,15-4,28 (m, 2 H, Tyr-^{α}CH-, Met-^{α}CH-), δ=5,65 (s, 2 H, AC-NH₂), δ=6,28 (d, 1 H, Tyr-^{α}NH-), δ=6,63 (d, 2 H, Tyr-ArH), δ=6,97 (d, 2 H, Tyr-ArH), δ=7,04 (s, 1 H, Met-NH₂), δ=7,25 (s, 1 H, Met-NH₂), δ=8,08 (d, 1 H, Met-^{α}NH-), δ=8,54 (s, 1 H, Tyr-OH)

### Beispiel 12: Synthese von AC-Tyr-Nvl-NH₂

Analog Beispiel 9a werden statt H-Ile-Ala-NH₂•HCl 31,2 mg (0.2 mmol) H-Hvl-NH₂•HCl eingesetzt.
Ausbeute an AC-Tyr-Nvl-NH₂ nach 14 h: 88 % d. Th.
¹H-NMR (D₆-DMSO, 300 MHz): δ=0,85 (t, 3 H, Nvl-^{δ}CH₃), δ=1,18-1,32 (m, 2 H, Nvl-^{γ}CH₂-), δ=1,45-1,68 (m, 2 H, Nvl-^{β}CH₂-), δ=2,55-2,68 (m, 1 H, Tyr-^{β}CH₂-), δ=2,78-2,88 (m, 1 H, Tyr-^{β}CH₂-), δ=4,12-4,30 (m, 2 H, Tyr-^{α}CH-, Nvl-αCH-), δ=5,59 (s, 2 H, AC-NH₂), δ=6,02 (d, 1 H, Tvr-^{α}NH-), δ=6,62 (d, 2 H, Tyr-ArH), δ=6,94-6,99 (m, 3 H, 2 Tyr-ArH, 1 Nvl-NH₂), δ=7,21 (s, 1 H, Nvl-NH₂), δ=7,79 (d, 1 H, Met-^{α}NH-), δ=9,12 (s, 1 H, Tyr-OH)

### Beispiel 13: Synthese von AC-Tyr-Phe-NH₂

Analog Beispiel 9a werden statt H-Ile-Ala-NH₂•HCl 40 mg (0,2 mmol) H-Phe-NH₂•HCl eingesetzt.
Ausbeute an AC-Tyr-Phe-NH₂ nach 1 h: 86 % d. Th.
¹H-NMR (D₆-DMSO, 300 MHz): δ=2,72-2,88 (m, 3 H, -^{β}CH₂-), δ=1,98-3,06 (m, 1 H, -^{β}CH₂-), δ=4,12-4,20 (m, 1 H, -^{α}CH-), δ=4,38-4,46 (m, 1 H, -^{α}CH-), δ=5,59 (s, 2 H, AC-NH₂), δ=5,97 (d, 1 H, Tyr-^{α}NH-), δ=6,61 (d, 2 H, Tyr-ArH), δ=6,92 (d, 2 H, Tyr-ArH), δ=7,08 (s, 1 H, Phe-NH₂), δ=7,15-7,28 (m, 5 H, Phe-C₆H₅), δ=7,32 (s, 1 H, Phe-NH₂) , δ=7,92 (d, 1 H, Phe-^{α}NH-), δ=9,12 (s, 1 H, Tyr-OH)

### Beispiel 14: Synthese von AC-Tyr-Phe-OMe

Analog Beispiel 9a werden statt H-Ile-Ala-NH₂•HCl 43 mg (0,2 mmol) H-Phe-OMe•HCl eingesetzt. Die verwendete Thermolysinmenge beträgt 2 mg.
Ausbeute an AC-Tyr-Phe-OMe nach 14 h: 81 % d. Th.
¹H-NMR (D₆-DMSO, 300 MHz): δ=2,50-2,62 (m, 1 H, -^{β}CH₂-), δ=2,72-2,85 (m, 1 H, -^{β}CH₂-), δ=2,90-3,08 (m, 2 H, -^{β}CH₂-), δ=3,58 (s, 3 H, -O-CH₃), δ=4,25-4,35 (m, 1 H, -^{α}CH-), δ=4,44-4,52 (m, 1 H, -^{α}CH-), δ=5,52 (s, 2 H, AC-NH₂), δ=5,99 (d, 1 H, Tyr-^{α}NH-), δ=6,62 (d, 2 H, Tyr-ArH), δ=6,94 (d, 2 H, Tyr-ArH), δ=7,15-7,30 (m, 5 H, Phe-C₆H₅), δ=8,36 (d, 1 H, Phe-^{α}NH-), δ=9,14 (s, 1 H, Tyr-OH)

### Beispiel 15: Synthese von AC-Tyr-Ile-NH₂

Analog Beispiel 9a werden statt H-Ile-Ala-NH₂•HCl 33,2 mg (0,2 mmol) H-Ile-NH₂•HCl eingesetzt.
Ausbeute an AC-Tyr-Ile-NH₂ nach 18 h: 68,9 % d. Th.
¹H-NMR (D₆-DMSO, 300 MHz): δ=0,76-0,86 (m, 6 H, Ile-^{β}CH₃, Ile-^{γ}CH₃), δ=0,99-1,12 (m, 1 H, Ile-^{γ}CH₂-), δ=1,35-1,46 (m, 1 H, Ile-^{γ} CH₂-), δ=1,62-1,76 (m, 1 H, Ile-^{β}CH-), δ=2,58-2,68 (m, 1 H, Tyr-^{β}CH₂-), δ=2,79-2,88 (m, 1 H, Tyr-^{β} CH₂-), δ=4,12 (dd, 1 H, Ile-^{α}CH-), δ=4,26-4,37 (m, 1 H, Tyr-^{α}CH-), δ=5,58 (s, 2 H, AC-NH₂), δ=6,06 (d, 1 H, Tyr-^{α}NH-), δ=6,62 (d, 2 H, Tyr-ArH), δ=6,96 (d, 2 H, Tyr-ArH), δ=7,02 (s, 1 H, Ile-NH₂), δ=7,31 (s, 1 H, Ile-NH₂), δ=7,68 (d, 1 H, Ile-^{α} NH-), δ=9,12 (s, 1 H, Tyr-OH)

### Beispiel 16: Synthese von AC-Tyr-Leu-NH₂

Analog Beispiel 9a werden 224 mg (1,0 mmol) AC-Tyr-OH, 130 mg (1.0 mmol) H-Leu-NH₂ (Bachem Feinchemikalien AG), 225 µl Puffer, keine NaOH und 50 µl Thermolysinsuspension, die 5 mg Thermolysin (Sigma P1512) enthalten, eingesetzt.
Ausbeute an AC-Tyr-Leu-NH₂ nach 16 h:
127,8 mg (= 38 % d. Th.)
¹H-NMR (D₆-DMSO, 300 MHz): δ=0,80-0,92 (m, 6 H, Leu-^{δ}CH₃), δ=1,42-1,62 (m, 3 H, Leu-^{γ} CH-, Leu-^{β}CH₂-) , δ=2,58-2,70 (m, 1 H, Tyr-^{β} CH₂-), δ=2,79-2,88 (m, 1 H, Tyr-^{β}CH₂-), δ=4,16-4,30 (m, 2 H, Leu-^{α}CH-, Tyr-^{α}CH-), δ=5,60 (s, 2 H, AC-NH₂), δ=6,02 (d, 1 H, Tyr-^{α}NH-), δ=6,63 (d, 2 H, Tyr-ArH), δ=6,93-7,00 (m, 2 H, Tyr-ArH, 1 H, Leu-NH₂), δ=7,19 (s, 1 H, Leu-NH₂), δ=7,83 (d, 1 H, Leu-^{α}NH-), δ=9,15 (s, 1 H, Tyr-OH)

### Beispiel 17: Synthese von AC-Tyr-Ile-Gly-OMe

Analog Beispiel 9a werden statt H-Ile-Ala-NH₂•HCl 47,8 mg (0,2 mmol) H-Ile-Gly-OMe•HCl eingesetzt.
Ausbeute an AC-Tyr-Ile-Gly-OMe nach 3 h: 89 % d. Th.
¹-NMR;COSY(D₆-DMSO, 300 MHz): δ=0,76-0,88 (m, 6 H, Ile-^{δ}CH₃, Ile-^{γ}CH₃), δ=1,00-1,14 (m, 1 H, Ile-^{γ}CH₂-), δ=1,36-1,52 (m, 1 H, Ile-^{γ}CH₂-), δ=1,65-1,78 (m, 1 H, Ile-^{β}CH-), δ=2,55-2,68 (m, 1 H, Tyr-^{β}CH₂-), δ=2,75-2,86 (m, 1 H, Tyr-^{β}CH₂-), δ=3,62 (s, 3 H, -O-CH₃), δ=3,84 (t, 2 H, Gly-^{α}CH₂-), δ=4,27-4,35 (m, 1 H, Ile-^{α}CH-), δ=4,38-4,48 (m, 1 H, Tyr-^{α} CH-), δ=5,57 (s, 2 H, AC-NH₂), δ=6,05 (d, 1 H, Tyr-^{α}NH-), δ=6,61 (d, 2 H, Tyr-ArH), δ=6,95 (d, 2 H, Tyr-ArH), δ=7,79 (d, 1 H, Ile-^{α}NH), δ=8,32 (t, 1 H, Gly-^{α}NH-), δ=9,11 (s, 1 H, Tyr-OH)

### Beispiel 18: Synthese von AC-Tyr-Val-OBzl

Analog Beispiel 16 werden statt H-Leu-NH₂ 379,4 mg (1.0 mmol) H-Val-OBzl•pTos (Bachem Feinchemikalien AG), 175 µl Puffer, 125 µL 10 M wäßrige NaOH und 100 µl Thermolysinsuspension, die 10 mg Thermolysin (Sigma P1512) enthalten, eingesetzt.
Ausbeute an AC-Tyr-Val-OBzl nach 88 h:
230 mg (= 55 % d. Th.)
¹H-NMR (D₆-DMSO, 300 MHz): δ=0,81-0,92 (m, 6 H, Val-^{γ}CH₃), δ=2,01-2,14 (m, 1 H, Val-^{β}CH-), δ=2,50-2,64 (m, 1 H, Tyr-^{β}CH₂-), δ=2,75-2,85 (m, 1 H, Tyr-^{β}CH₂-), δ=4,23 (dd, 1 H, Val-^{α}CH-), δ=4,36-4,45 (m, 1 H, Tyr-^{α}CH-), δ=5,13 (s, 2 H, -O-CH₂-), δ=5,52 (s, 2 H, AC-NH₂), δ=6,04 (d, 1 H, Tyr-^{α}NH-), δ=6,62 (d, 2 H, Tyr-ArH), δ=6,96 (d, 2 H, Tyr-ArH), δ=7,30-7,40 (m, 5 H, -C₆H₅), δ=8,20 (d, 1 H, Val-^{α}NH-), δ=9,13 (s, 1 H, Tyr-OH)

### Beispiel 19: Synthese von AC-Tyr-Phe-Ala-OBzl

Analog Beispiel 9a werden statt H-Ile-Ala-NH₂•HCl 88 mg (0,2 mmol) H-Phe-Ala-OBzl•TFA eingesetzt.
Ausbeute an AC-Tyr-Phe-Ala-OBzl nach 3 h: 72 % d. Th.
¹H-NMR;COSY(D₆-DMSO, 300 MHz): δ=1,33 (d, 3 H, Ala-^{β}CH₃), δ=2,48-2,58 (m, 1 H, Tyr-^{β}CH₂-), δ=2,70-2,85 (m, 2 H, Tyr-^{β}CH₂-), δ=2,95-3,05 (m, 1 H, Phe-^{β}CH₂-), δ=4,14-4,24 (m, 1 H, Tyr-^{α}CH-), δ=4,32-4,41 (m, 1 H, Ala-^{α}CH-), δ=4,51-4,62 (m, 1 H, Phe-^{α}CH-), δ=5,13 (s, 2 H, O-CH₂-), δ=5,56 (s, 2 H, AC-NH₂), δ=5,94 (d, 1 H, Tyr-^{α}NH-), δ=6,60 (d, 2 H, Tyr-ArH), δ=6,90 (d, 2 H, Tyr-ArH), δ=7,14-7,28 (m, 5 H, OBzl-C₆H₅), δ=7,30-7,40 (m, 5 H, Phe-C₆H₅), δ=8,01 (d, 1 H, Phe-^{α}NH), δ=8,46 (d, 1 H, Ala-^{α}NH-), δ=9,12 (s, 1 H, Tyr-OH)

### Beispiel 20: Synthese von AC-Met-Phe-NH₂

Analog Beispiel 9a werden statt AC-Tyr-OH 76,8 mg (0,2 mmol) AC-Met-OH 80 mg (0,4 mmol) H-Phe-NH₂•HCl (Degussa AG), 90 µl Puffer, 50 µl 10 M wäßrige NaOH und 20 µl Thermolysinsuspension, die 2 mg Thermolysin (Sigma P1512) enthalten, eingesetzt.
Ausbeute an AC-Met-Phe-NH₂ nach 3 h: 96 % d. Th.
¹H-NMR (D₆-DMSO, 300 MHz): δ=1,58-1,80 (m, 2 H, Met-^{β}CH₂-), δ=1,99 (s, 3 H, Met-S-CH₃), δ=2,25-2,36 (m, 2 H, Met-^{γ}CH₂-), δ=2,76-2,88 (m, 1 H, Phe-^{β}CH₂-), δ=2,98-2,08 (m, 1 H, Phe-^{β} CH₂-), δ=3,98-4,08 (m, 1 H, -^{α}CH-) , δ=4,36-4,47 (m, 1 H, -^{α}CH-), δ=5,63 (s, 2 H, AC-NH₂), δ=6,23 (d, 1 H, Met-^{α}NH-), δ=7,09 (s, 1 H, Phe-NH₂) , δ=7,12-7,28 (m, 5 H, Phe-C₆H₅), δ=7,42 (s, 1 H, Phe-NH₂), δ=7,96 (d, 1 H, Phe-^{α}MH-)

### Beispiel 21: Synthese von AC-Leu-Phe-NH₂

Analog Beispiel 20 werden statt AC-Met-OH 69,6 mg (0,4 mmol) AC-Leu-OH eingesetzt.
Ausbeute an AC-Leu-Phe-NH₂ nach 70 h: 97 % d. Th.
¹H-NMR (D₆-DMSO, 300 MHz): δ=0,78-0,88 (m, 6 H, Leu-^{δ}CH₂-), δ=1,16-1,35 (m, 2 H, Leu-^{β}CH₂-, Leu-^{γ}CH-), δ=1,45-1,56 (m, 1 H, Leu-^{β}CH₂-), δ=2,78-2,90 (m, 1 H, Phe-^{β}CH₂-), δ=2,99-3,08 (m, 1 H, Phe-^{β}CH₂-), δ=3,90-4,00 (m, 1 H, -^{α} CH-), δ=4,36-4,45 (m, 1 H, -^{α}CH-), δ=5,58 (s, 2 H, AC-NH₂), δ=6,08 (d, 1 H, Leu-^{α} NH-), δ=7,06 (s, 1 H, Phe-NH₂), δ=7,12-7,28 (m, 5 H, Phe-C₆H₅), δ=7,34 (s, 1 H, Phe-NH₂), δ=7,82 (d, 1 H, Phe-^{α}NH-)

### Beispiel 22

### Abspaltung der Carbamoyl-Schutzgruppe (exemplarisch am Beispiel von Carbamoyl-Aspartam) mittels Enzymtechnik

16 ml (65,6 U) Carbamoylase und 50,6 mg Thermolysin werden gemeinsam in Wasser vorgelegt und über Nacht (ca. 18 h) gerührt. Das Carbamoyl-Aspartam (470 mg), 1,25 mmol) wird zugegeben. Gesamtvolumen: = 16 ml. Der pH-Wert liegt zwischen 6,5 und 7,0. Nach 24 h ist kein Carbamoyl-Aspartam mehr nachzuweisen. Laut HPLC-Eichung ist der Umsatz zu Aspartam 98,3 %.

### Beispiel 23

### Abspaltung der Carbamoyl-Schutzgruppe (exemplarisch am Beispiel von Carbamoyl-L-Aspartam) durch Zusatz von Natriumnitrit

4.72 g (12.6 mmol) Carbamoyl-L-Aspartam-Kaliumsalz werden in 60 ml H₂O / 40 ml HCl (konz., technisch) vorgelegt, auf 5 °C abgekühlt (pH = 0.80) und 15.1 ml (15.1 mmol) NaNO₂-Lösung langsam (1 ml/10 min) zudosiert. Man läßt ca. 1 h nachreagieren, bevor das Reaktionsgemisch mit 50 Gew.-% Natronlauge neutralisiert wird. Das Reaktionsgemisch wird über Nacht bei RT belassen, bevor der ausgefallene Niederschlag abgetrennt und verworfen wird. Die Mutterlauge wird anschließend eingeengt bis Aspartam ausfällt. Das so gewonnene Aspartam wird im Vakuum bei 60 °C bis zur Gewichtskonstanz getrocknet. Die Umsetzung verläuft quantitativ.

## Patentansprüche

1. Verfahren zur Herstellung von Peptiden der allgemeinen Struktur I, 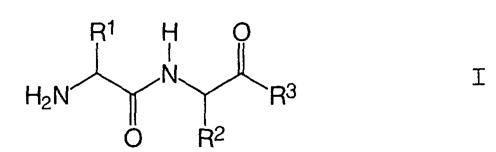 worin
R¹ und R² unabhängig voneinander Wasserstoff, (C₁-C₆) Alkyl, das ggf. ein oder mehrfach mit Heteroatome, wie N, O oder S, unterbrochen oder substituiert sein kann, wobei die Heteroatome ihrerseits mit Wasserstoff, (C₁-C₄) Alkyl oder Benzyl substituiert sein können oder über eine Doppelbindung mit der Alkylgruppe verbunden sein können, Phenyl oder Benzyl, die beide ggf. ein- oder mehrfach mit Halogen oder Hydroxy substituiert sein können, Heteroaralkyl, wie 3-Indolylmethyl, 2-, 3- oder 4-Pyridylmethyl,
R³ (C₁-C₄) Alkoxy, NH₂, Hydroxy, NF¹R², Benzyloxy, das ggf. ein- oder mehrfach durch Halogen, Nitro, NH₂, (C₁-C₄) Alkyl, (C₁-C₄) Alkoxy substituiert sein kann, oder eine oder mehrere Einheiten des Typs II 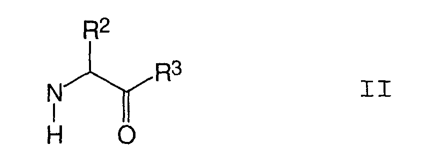 bedeuten,
**dadurch gekennzeichnet,**
daß man Verbindungen des Typs III oder eine Salzform davon, 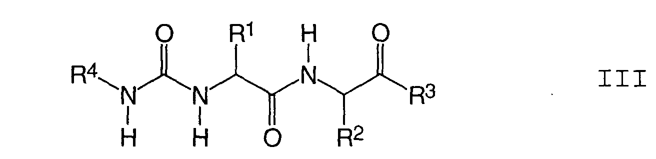 worin R¹, R² und R³ die oben angegebene Bedeutung besitzen und
R⁴ Wasserstoff, (C₁-C₄) Alkyl, Phenyl, das ggf. ein- oder mehrfach mit Halogen, (C₁-C₄) Alkyl, (C₁-C₄) Alkoxy, Nitro, CN, CF₃,
(C₁-C₆) Alkoxycarbonyl, COOH oder -NR¹R² substituiert sein kann, Aralkyl, wie Benzyl, das seinerseits mit Halogen, (C₁-C₄) Alkyl oder (C₁-C₄) Alkoxy substituiert sein kann, Naphthyl, Heteroaralkyl, wie 2-, 3- oder 4-Thienyl, 2-, 3- oder 4-Pyridyl oder 2-Chinolyl
bedeutet,
zur Abspaltung der Carbamoyl-Schutzgruppe mit einer Carbamoylase, wahlweise in Gegenwart eines Lösungsmittels, zur Reaktion bringt.

2. Verfahren nach Anspruch 1,
**dadurch gekennzeichnet,**
daß die Peptide der allgemeinen Struktur III, 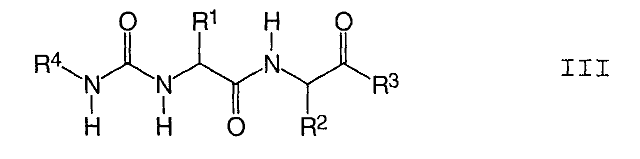 worin
R¹ und R² unabhängig voneinander Wasserstoff, (C₁-C₆) Alkyl, das ggf. ein oder mehrfach mit Heteroatome, wie N, O oder S, unterbrochen oder substituiert sein kann, wobei die Heteroatome ihrerseits mit Wasserstoff, (C₁-C₄) Alkyl oder Benzyl substituiert sein können oder über eine Doppelbindung mit der Alkylgruppe verbunden sein können, Phenyl oder Benzyl, die beide ggf. ein- oder mehrfach mit Halogen oder Hydroxy substituiert sein können, Heteroaralkyl, wie 3-Indolylmethyl, 2-, 3- oder 4-Pyridylmethyl,
R³ (C₁-C₄) Alkoxy, NH₂, Hydroxy, NR¹R², Benzyloxy, das ggf. ein- oder mehrfach durch Halogen, Nitro, NH₂, (C₁-C₄) Alkyl, (C₁-C₄) Alkoxy substituiert sein kann,
R⁴ Wasserstoff, (C₁-C₄) Alkyl, Phenyl, das ggf. ein- oder mehrfach mit Halogen, (C₁-C₄) Alkyl, (C₁-C₄) Alkoxy, Nitro, CN, CF₃, (C₁-C₆) Alkoxycarbonyl, COOH oder -NR¹R² substituiert sein kann, Aralkyl, wie Benzyl, das seinerseits mit Halogen, (C₁-C₄) Alkyl oder (C₁-C₄) Alkoxy substituiert sein kann, Naphthyl, Heteroaralkyl, wie 2-, 3- oder 4-Thienyl, 2-, 3- oder 4-Pyridyl oder 2-Chinolyl
bedeuten, aus einer Verbindung des Typs IV oder einer Salzform von IV, in der R¹ und R⁴ die oben angegebene Bedeutung besitzen, 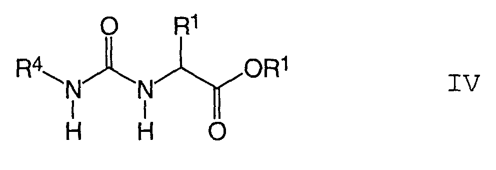 in Gegenwart von Hydrolasen, wahlweise in Gegenwart eines Lösungsmittels und wahlweise in Gegenwart einer Base, und einer Verbindung des Typs V oder eines Säureadditionssalzes hiervon, worin R² und R³ die oben angegebene Bedeutung besitzen, 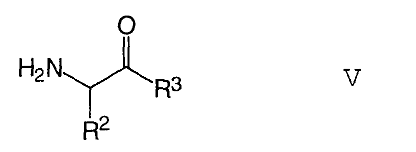 hergestellt wird.

3. Verfahren nach Anspruch 1,
**dadurch gekennzeichnet,**
daß die Umsetzung mit Carbamoylase in einem Temperaturbereich zwischen 10 °C und 50 °C, vorzugsweise 20 °C und 35 °C, ganz besonders bevorzugt zwischen 25 °C und 30 °C, erfolgt.

4. Verfahren nach Anspruch 1,
**dadurch gekennzeichnet,**
daß die Umsetzung mit Carbamoylase in einem pH-Bereich zwischen 5 und 11, bevorzugt zwischen 6,5 und 8, erfolgt.

5. Verfahren nach einem der vorhergehenden Ansprüche,
**dadurch gekennzeichnet,**
daß die Carbamoylase aus den Mikroorganismen DSM 7329, DSM 7330 oder DSM 9771 stammt.

6. Verfahren nach einem der vorangegangenen Ansprüche,
**dadurch gekennzeichnet,**
daß die Carbamoylase ggf. in teilgereinigtem oder gereinigtem, isoliertem oder immobilisiertem Zustand eingesetzt wird.

7. Verfahren nach Anspruch 3,
**dadurch gekennzeichnet,**
daß die Umsetzungen im wäßrigen Medium stattfinden.

8. Verfahren nach einem der vorangegangenen Ansprüche,
**dadurch gekennzeichnet,**
daß das gespaltene Peptidderivat Aspartam ist.

9. Verfahren nach Anspruch 2,
**dadurch gekennzeichnet,**
daß die Peptidkopplung in hochdichter Suspension erfolgt.

10. Verfahren nach Anspruch 9,
**dadurch gekennzeichnet,**
daß Substrat und Produkt in teilweise fester Form vorliegen.

## Claims

1. Process for the preparation of peptides of the general structure I, 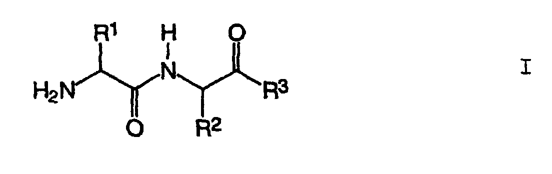 wherein
R¹ and R² independently of one another denote hydrogen, (C₁-C₆) alkyl, which can optionally be interrupted or substituted by heteroatoms, such as N, O or S, one or several times, it being possible for the heteroatoms in their turn to be substituted by hydrogen, (C₁-C₄) alkyl or benzyl or to be bonded to the alkyl group via a double bond, phenyl or benzyl, both of which can optionally be substituted by halogen or hydroxyl once or several times, heteroaralkyl, such as 3-indolylmethyl, 2-, 3- or 4-pyridylmethyl,
R³ denotes (C₁-C₄) alkoxy, NH₂, hydroxyl, NR¹R², benzyloxy, which can optionally be substituted by halogen, nitro, NH₂, (C₁-C₄) alkyl, (C₁-C₄) alkoxy once or several times, or one or more units of the type II 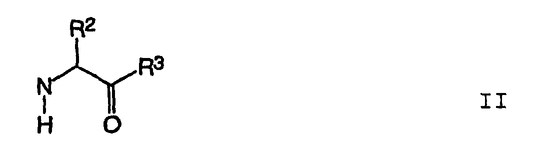 characterized in that
compounds of the type III or a salt form thereof, 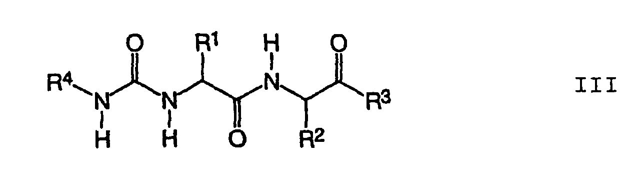 wherein R¹, R² and R³ have the abovementioned meaning and
R⁴ denotes hydrogen, (C₁-C₄) alkyl, phenyl, which can optionally be substituted by halogen, (C₁-C₄) alkyl, (C₁-C₄) alkoxy, nitro, CN, CF₃, (C₁-C₆) alkoxycarbonyl, COOH or -NR¹R² once or several times, aralkyl, such as benzyl, which can be substituted in its turn by halogen, (C₁-C₄) alkyl or (C₁-C₄) alkoxy, naphthyl, heteroaralkyl, such as 2-, 3- or 4-thienyl, 2-, 3- or 4-pyridyl or 2-quinolyl
are reacted with a carbamoylase, optionally in the presence of a solvent, to split off the carbamoyl protective group.

2. Process according to claim 1,
characterized in that
the peptides of the general structure III, 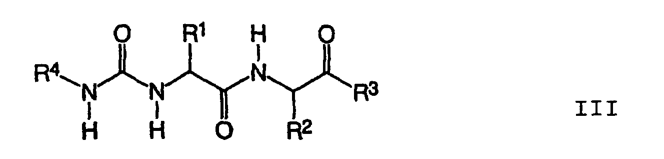 wherein
R¹ and R² independently of one another denote hydrogen, (C₁-C₆) alkyl, which can optionally be interrupted or substituted by heteroatoms, such as N, O or S, one or several times, it being possible for the heteroatoms in their turn to be substituted by hydrogen, (C₁-C₄) alkyl or benzyl or to be bonded to the alkyl group via a double bond, phenyl or benzyl, both of which can optionally be substituted by halogen or hydroxyl once or several times, heteroaralkyl, such as 3- indolylmethyl, 2-, 3- or 4-pyridylmethyl,
R³ denotes (C₁-C₄) alkoxy, NH₂, hydroxyl, NR¹R², benzyloxy, which can optionally be substituted by halogen, nitro, NH₂, (C₁-C₄) alkyl, (C₁-C₄) alkoxy once or several times,
R⁴ denotes hydrogen, (C₁-C₄) alkyl, phenyl, which can optionally be substituted by halogen, (C₁-C₄) alkyl, (C₁-C₄) alkoxy, nitro, CN, CF₃, (C₁-C₆) alkoxycarbonyl, COOH or -NR¹R² once or several times, aralkyl, such as benzyl, which can be substituted in its turn by halogen, (C₁-C₄) alkyl or (C₁-C₄) alkoxy, naphthyl, heteroaralkyl, such as 2-, 3- or 4-thienyl, 2-, 3- or 4-pyridyl or 2-quinolyl
is prepared from a compound of the type IV or a salt form of IV, in which R¹ and R⁴ have the abovementioned meaning, 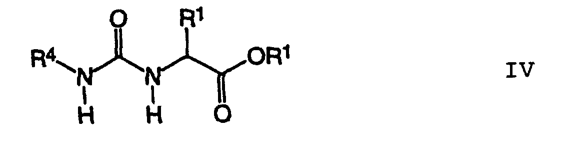 in the presence of a hydrolase, optionally in the presence of a solvent and optionally in the presence of a base, and a compound of the type V or of an acid addition salt thereof, wherein R² and R³ have the abovementioned meaning

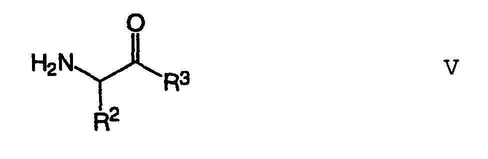

3. Process according to claim 1,
characterized in that,
the reaction with carbamoylase is carried out in a temperature range between 10 °C and 50 °C, preferably 20 °C and 35 °C, very particularly preferably between 25 °C and 30 °C.

4. Process according to claim 1,
characterized in that
the reaction with carbamoylase is carried out in a pH range between 5 and 11, preferably between 6.5 and 8.

5. Process according to one of the preceding claims,
characterized in that
the carbamoylase originates from the microorganisms DSM 7329, DSM 7330 or DSM 9771.

6. Process according to one of the preceding claims,
characterized in that
the carbamoylase is employed optionally in a partly purified or purified, isolated or immobilized state.

7. Process according to claim 3,
characterized in that
the reactions take place in an aqueous medium.

8. Process according to one of the preceding claims,
characterized in that
the peptide derivative cleaved is aspartam.

9. Process according to claim 2,
characterized in that
the peptide coupling is carried out in a highly dense suspension.

10. Process according to claim 9,
characterized in that
the substrate and product are present in partly solid form.

## Revendications

1. Procédé de production de peptides de structure générale I, 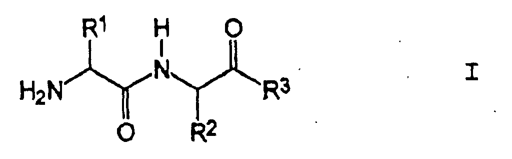 dans laquelle
- R¹ et R² représentent indépendamment l'un de l'autre, un hydrogène, un alkyle en C₁ à C₆, - qui peut être interrompu ou substitué le cas échéant une ou plusieurs fois par des hétéroatomes comme N, O ou S, les hétéroatomes de leur côté pouvant être substitués par l'hydrogène, un alkyle en C₁ à C₄ ou un benzyle, ou pouvant être liés par l'intermédiaire d'une double liaison avec le groupe alkyle -, un phényle ou un benzyle, - qui l'un et l'autre peuvent être le cas échéant une ou plusieurs fois substitués par un halogène ou un hydroxy -,un hétéro-aralkyle, comme le 3-indolyl-méthyle, le 2-, 3- ou 4-pyridylméthyle,
- R³ représente un alcoxy en C₁ à C₄, NH₂, un hydroxy, NR¹R², un benzyloxy, - qui le cas échéant peut être une ou plusieurs fois substitué par un halogène, un nitro, NH₂, un alkyle en C₁ à C₄, un alcoxy en C₁ à C₄,
ou représente une ou plusieurs unités de type II 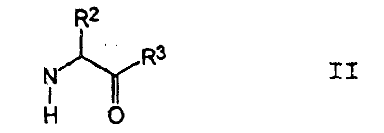 caractérisé en ce que
on fait réagir des composés de type III ou une de leurs formes sel 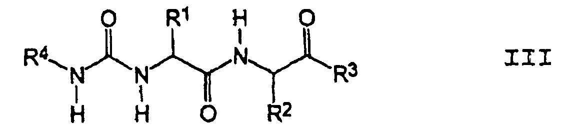 dans laquelle R¹, R² et R³ ont la signification indiquée ci-dessus, et
- R⁴ représente un hydrogène, un alkyle en C₁ à C₄, un phényle, - qui le cas échéant peut être une ou plusieurs fois substitué par un halogène, un alkyle en C₁ à C₄, un alcoxy en C₁ à C₄, un nitro, CN, CF₃, un alcoxy en C₁ à C₆-carbonyle, COOH ou -NR¹R² -, un aralkyle, comme le benzyle, - qui de son côté peut être substitué par un halogène, un alkyle en C₁ à C₄ ou un alcoxy en C₁ à C₄ -, - un naphtyle, un hétéroalkyle, comme le 2-, 3- ou 4-thiényle, le 2-, 3- ou 4-pyridyle ou le 2-quinolyle,
pour séparer le groupe protecteur carbamoyle, avec une carbamoylase, au choix, en présence d'un solvant.

2. Procédé selon la revendication 1,
caractérisé en ce qu'
on produit les peptides de structure générale III protégés par un carbamoyle 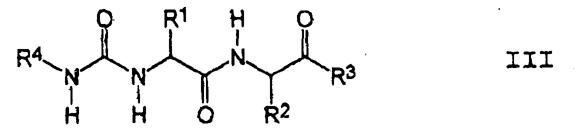 dans laquelle
- R¹ et R² représentent indépendamment l'un de l'autre un hydrogène, un alkyle en C₁ à C₆, - qui le cas échéant peut être interrompu substitué une ou plusieurs fois par des hétéroatomes, comme N, O ou S, les hétéroatomes de leur côté pouvant être substitués par l'hydrogène, un alkyle en C₁ à C₄ ou le benzyle, ou liés par l'intermédiaire d'une double liaison avec le groupe alkyle -, un phényle ou un benzyle, - qui l'un et l'autre peuvent être le cas échéant une ou plusieurs fois substitués par un halogène ou un hydroxy -, un hétéroaralkyle, comme le 3-indolyle méthyle, le 2-, 3- ou 4-pyridylméthyle,
- R³ représente un alcoxy en C₁ à C₄, NH₂, un hydroxy, NR¹R², un benzyloxy, qui le cas échéant peut être une ou plusieurs fois substitué par un halogène, un nitro, NH₂, un alkyle en C₁ à C₄, un alcoxy en C₁ à C₄,
- R⁴ représente un hydrogène, un alkyle en C₁ à C₄, un phényle, - qui le cas échéant peut être une ou plusieurs fois substitué par un halogène, un alkyle en C₁ à C₄, un alcoxy en C₁ à C₄, un nitro, CN, CF₃, un alcoxy en C₁ à C₆ carbonyle, COOH ou -NR¹R²-, un aralkyle, comme le benzyle, - qui de son côté peut être substitué par un halogène, un alkyle en C₁ à C₄ ou un alcoxy en C₁ à C₄₋-, un naphtyle, un hétéroaralkyle, comme le 2-, 3- ou 4-thinyle, le 2-, 3- ou 4-pyridyle ou 2-quinoléyle,
en préparant à partir d'un composé de type IV ou d'une forme sel de IV, dans laquelle R¹ et R⁴ ont la signification indiquée ci-dessus, 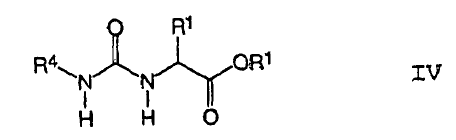 en présence d'hydrolases, au choix en présence d'un solvant et au choix en présence d'une base, et d'un composé de type V, ou d'un de ses sels d'addition d'acides, où R² et R³ ont la signification indiquée ci-dessus

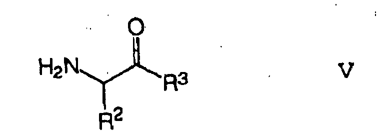

3. Procédé selon la revendication 1,
caractérisé en ce qu'
on conduit la réaction avec une carbamoylase dans un intervalle de température allant de 10°C à 50°C, de préférence de 20°C à 35°C, de façon tout particulièrement préférée de 25°C à 30°C.

4. Procédé selon la revendication 1,
caractérisé en ce qu'
on conduit la réaction avec une carbamoylase dans un intervalle de pH allant de 5 à 11, de préférence de 6,5 à 8.

5. Procédé selon l'une des revendications précédentes,
caractérisé en ce que
la carbamoylase provient des microorganismes DSM 7329, DSM 7330 ou DSM 9771.

6. Procédé selon l'une des revendications précédentes,
caractérisé en ce qu'
on utilise la carbamoylase le cas échéant sous une forme partiellement purifiée ou purifiée, isolée ou immobilisée.

7. Procédé selon la revendication 3,
caractérisé en ce que
les transformations se déroulent en milieu aqueux.

8. Procédé selon l'une des revendications précédentes,
caractérisé en ce que
le dérivé peptidique séparé est l'aspartame.

9. Procédé selon la revendication 2,
caractérisé en ce que
le couplage peptidique s'effectue dans une suspension très dense.

10. Procédé selon la revendication 9,
caractérisé en ce que
le substrat et le produit se présentent sous une forme en partie solide.
